# EUROPÄISCHE PATENTANMELDUNG

(11) **EP 1 832 333 A1**
(43) Veröffentlichungstag der Anmeldung: **12.09.2007**
(21) Anmeldenummer: 07103973.9
(22) Anmeldetag: 12.03.2007
(51) Int. Cl.: B01D 61/14, B01D 61/16, B01D 61/22, B23Q 11/10, C02F 9/00, C07H 1/06, C10M 175/04, C13K 1/08

(54) **Verfahren zur Wiedergewinnung von verunreinigten Fluiden**

(30) Priorität: 10.03.2006 AT 3972006
(71) Anmelder: Seiler Verfahrenstechnik GmbH, 2540 Bad Vöslau (AT)
(72) Erfinder: Arnberger, Peter, 2384, Breitenfurt (AT); Joksch, Martin, 2500, Baden (AT)
(74) Vertreter: Müllner, Martin

(57) **Zusammenfassung**

Verfahren zur Wiedergewinnung von mit Fremdstoffen beladenen wässrigen, neutralen, alkalischen, sauren oder enzymatischen Fluiden, z.B. aus der zerspanenden Bearbeitung von Metallen und Kunststoffen, oder von mit Farben und Lacken verunreinigten Lösungen oder von mit organischen Substanzen, wie z.B. Stärke, verunreinigten Fluiden, bei welchem Verfahren das mit Fremdstoffen beladene Fluid einer Querstromfiltration unterworfen wird. Um eine einfache Durchführung eines solchen Verfahrens und einen hohen Trocknungsgrad des Konzentrats zu erreichen, ist vorgesehen, dass das bei der Filtration gewonnene Konzentrat durch Zentrifugieren auf einen höheren Gehalt an Trockensubstanz gebracht wird und das Permeat der Filtration einer Wiederverwertung zurückgeführt wird.

## Beschreibung

### Technisches Gebiet

Die Erfindung bezieht sich auf ein Verfahren zur Wiedergewinnung von mit Fremdstoffen verunreinigten Fluiden gemäß dem Oberbegriff des Anspruchs 1.

### Stand der Technik

Ein solches Verfahren wird zur Wiedergewinnung von Arbeits- und Reinigungslösungen benötigt, im Besonderen wässrig alkalischen und wässrigen Kühl- und Schmierflüssigkeiten beim Bearbeiten und Herstellen von Bauteilen, wie z.B. Sägewasser, der Wiedergewinnung von Spülwasser in der Lebensmittelindustrie (z.B. beim Waschen von Karotten und Kartoffeln) und der Rückführung von industriellen wässrigen Lösungen im Bereich von Druckereibetrieben, Farben und Textilveredelung, wo eine Verminderung der entsorgungspflichtigen Konzentratmenge gegenüber herkömmlichen Verfahren angestrebt wird. Dabei kann auch ein quasi trockenes Konzentrat von Farbpartikeln und/oder Inhaltsstoffen erreicht werden, wenn dies zur Ermöglichung einer Wiederverwendung gewünscht wird.

Derzeit werden solche wässrigen Lösungen und Flüssigkeiten in verschiedenen Arten filtriert und nur teilweise rückgewonnen, wobei die Filtration vom einfachen Sieb bis zur Nanofiltration reichen kann und anschließend keine weitere Behandlung oder sehr energieaufwändige Verfahren zur Verbesserung eingesetzt werden, wie Destillation, Vakuumdestillation oder auch chemieintensive, wie Fällung, mit dem Nachteil eines permanenten Abfallstroms oder eines hohen Energieeinsatzes und damit hoher Betriebskosten. Im Falle einer Fällung ergibt sich auch der Nachteil, dass sich die Zusammensetzung des Fluids nachhaltig ändert und eine Wiederverwertung desselben nicht möglich ist.

Der Wasser-, Energie- und/oder Chemikalienverbrauch ist somit sehr hoch, da eben nur ein Teil rückgewonnen wird und mit hohen Abfallströmen oder Energiekosten zu rechnen ist.

Ein Verfahren, bei dem Filtration mit Vakuumdestillation kombiniert wird, wurde z.B. durch die AT 408544 Bbekannt. Bei diesem bekannten Verfahren wird das Konzentrat aus der Ultrafiltrationsanlage einer Vakuum-Destillationsanlage zugeführt und so das Konzentrat eingedickt. Das abgedampfte Fluid wird dabei einem Lagerbehälter für das rückgewonnene Fluid zugeführt. Dabei ist auch vorgesehen, die Vakuum-Destillationsanlage mit einem Entschäumer zu beaufschlagen.

Bei diesem bekannten Verfahren ist es zwar möglich, eine erhebliche Reduzierung des Fluidgehaltes des Konzentrats zu erreichen. Allerdings ist bei der Durchführung dieses bekannten Verfahrens nicht nur ein sehr erheblicher apparativer Aufwand erforderlich, sondern auch ein sehr erheblicher Energieaufwand, sodass dieses Verfahren - soweit ersichtlich - in der Praxis noch nie erfolgreich eingesetzt wurde.

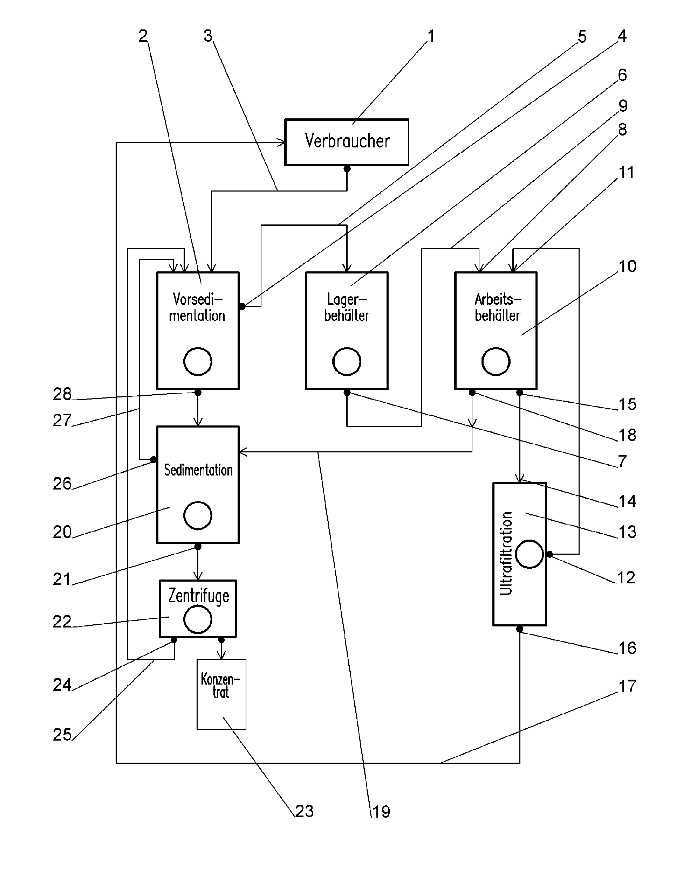

Ein Verfahren der eingangs genannten Art ist der US 2004161935 A andeutungsweise zu entnehmen. In den Absätzen [0015]-[0016] wird an Hand der Fig. 13 ein Verfahren erklärt, das damals schon Stand der Technik gewesen sein soll. Verunreinigtes Fluid wird von einem Schmutzwassertank 201 einer Filteranlage 203 zugeführt. Das gefilterte Wasser wird einem Klarwassertank 205 zugeführt und kann wieder verwendet werden. Sobald die Filter verstopft sind, wird rückgespült: Wasser vom Klarwassertank 205 wird über die Filteranlage 203 entgegen der normalen Filtrationsrichtung in den Schmutzwassertank 201 gepumpt. Dadurch gelangt hochgradig verschmutztes Wasser in den Schmutzwassertank 201. An dieser Stelle ist nun ein Fehler in der Beschreibung und in der Fig. 13 vorhanden, denn es heißt: Wasser aus dem Konzentrat-Wassertank 206 wird einer Zentrifuge 209 zugeführt - es ist aber nicht klar, wie das Konzentrat - das dem Schmutzwassertank zugeführt wird - in den Konzentrat-Wassertank gelangen soll. Auch die Fig. 13 ist diesbezüglich falsch, denn der Zulauf des Konzentrat-Wassertanks beginnt an einer Stelle der Filtrationsanlage, wo sich das Filtrat - also das gereinigte Wasser - befindet. Auf diese Weise kann kein Konzentrat in den Konzentrat-Wassertank 206 gelangen.

Jedenfalls ist klar, dass hier keine kontinuierlich arbeitenden Filter verwendet werden, weil rückgespült werden muss; das beim Rückspülen anfallende Konzentrat wird mit einer Zentrifuge weiter aufkonzentriert. Mit Filtern, die verstopfen und rückgespült werden müssen, werden aber keine guten Ergebnisse erzielt.

### Darstellung der Erfindung

### Technische Aufgabe

Ziel der Erfindung ist es, ein Verfahren der eingangs erwähnten Art vorzuschlagen, das effizient ist, sich mit relativ geringem apparativem Aufwand praktisch durchführen lässt und bei dem auch der Energieaufwand gering gehalten werden kann.

### Technische Lösung

Erfindungsgemäß wird dies bei einem Verfahren der eingangs erwähnten Art durch die kennzeichnenden Merkmale des Anspruchs 1 erreicht.

Durch die vorgeschlagenen Maßnahmen ist es auf einfache Weise möglich, das bei der Filtration gewonnene Konzentrat einzudicken. Man führt das Fluid im Arbeitsbehälter über eine Querstromfiltration im Kreis, bis das Fluid ausreichend eingedickt wird. Das danach anschließende Zentrifugieren ermöglicht, mit einer einfachen Apparatur - die überdies bei vielen derartigen Anlagen ohnehin schon vorhanden ist - das aufkonzentrierte Fluid einzudicken, wobei nur ein geringer Energieeinsatz erforderlich ist. Da außerdem der Klarablauf beim Zentrifugieren wieder der Ultrafiltration zugeführt wird und das Permeat der Ultrafiltration wieder verwendbar ist, ergibt sich z.B. bei wässrigen Lösungen eine erhebliche Verminderung des Frischwasserbedarfs.

Wenn man die Merkmale von Anspruch 2 vorsieht, ergibt sich der Vorteil, dass das Aufkonzentrieren des Fluids im Arbeitsbehälter nicht durch die Zufuhr von weiterer zu reinigender Flüssigkeit (z.B. aus der Filtration) gestört wird. Das zu reinigende Fluid wird zunächst in einem Lagerbehälter gesammelt und dem Arbeitsbehälter erst dann zugeführt, wenn das Fluid im Arbeitsbehälter fertig aufkonzentriert ist und der Zentrifuge zugeführt wurde.

Um den Einsatz von Materialien zu verringern, können die Merkmale des Anspruchs w vorgesehen werden.

Bei Fluiden, die mit organischem Kohlenstoff beaufschlagt sind, ist es vorteilhaft, die Merkmale des Anspruchs 4 vorzusehen.

In der Lebensmittelindustrie ist es vorteilhaft, die Merkmale des Anspruchs 5 vorzusehen. Dadurch können die im Konzentrat enthaltenen Stoffe, z.B. Stärke, wie sie im Waschwasser von Kartoffeln enthalten ist, risikolos verwertet werden.

Durch die Maßnahmen gemäß Anspruch 6 ergibt sich der Vorteil einer höheren Anreicherung des Konzentrats, bevor dieses zentrifugiert wird, sodass die Zentrifuge besser ausgenützt wird (sie wird mit weniger Masse beladen).

Durch die Merkmale des Anspruchs 7 ergibt sich der Vorteil, dass die Fremdstoffe aus dem Fluid sehr weitgehend rückgewonnen werden können und andererseits das Fluid praktisch vollständig rückgewonnen werden kann, wodurch auch kaum Fluidverluste auftreten. Außerdem wird die (Ultra-)filtrationsanlage insofern entlastet, als ein Teil der Feststoffe bereits zuvor absedimentiert wird.

Ein weiteres Ziel der Erfindung ist es, eine Einrichtung zur Durchführung des erfindungsgemäßen Verfahrens vorzuschlagen, die sich durch einen einfachen Aufbau auszeichnet.

Erfindungsgemäß werden daher ausgehend von einer Einrichtung gemäß dem Oberbegriff des Anspruchs 8 die kennzeichnenden Merkmale des Anspruchs 8 vorgeschlagen.

Durch diese Maßnahmen ist es auf einfache Weise möglich, das bei der Filtration gewonnene Konzentrat einzudicken und dessen Fluidgehalt zu reduzieren. Durch die Führung des Kreislaufs zwischen Ultrafiltrationsanlage und Arbeitsbehälter wird die Fremdstoffkonzentration im Arbeitsbehälter soweit erhöht, dass die Zentrifuge ausreichend Material abtrennen kann (und somit effizient genützt wird).

Um die Effizienz der Einrichtung zu steigern, ist es zweckmäßig, die Merkmale des Anspruchs 9 vorzusehen. Auf diese Weise wird die Zentrifuge mit Konzentrat beladen, dessen Fluidgehalt durch die Sedimentation vermindert ist, wodurch das Konzentrat mittels der Zentrifuge rascher auf den gewünschten Feststoffgehalt gebracht werden kann.

Um einen weitgehend geschlossenen Kreislauf des Fluids über den Verbraucher zu ermöglichen, ist es vorteilhaft, die Merkmale des Anspruchs 10 vorzusehen.

Durch diese Maßnahmen wird auch die Gewinnung der Feststoffe mit einem relativ geringen Energieaufwand ermöglicht, weil die (Ultra-)Filtrationsanlage entlastet wird.

### Kurze Beschreibung der Abbildungen der Zeichnungen

Die Erfindung wird nun anhand der Zeichnung näher erläutert, die schematisch eine erfindungsgemäße Einrichtung zeigt.

### Bester Weg zur Ausführung der Erfindung

Ein Verbraucher 1, z.B. eine spanabhebende Werkzeugmaschine mit Kühlmittelzufuhr, oder eine Waschanlage, z.B. für Kartoffel, oder eine Druckmaschine ist mit einem Vorsedimentationsbehälter 2 über eine Leitung 3 verbunden. Ein Klarablauf 4 des Vorsedimentationsbehälters 2 ist über eine Leitung 5 mit einem Lagerbehälter 6 verbunden, dessen Ablauf 7 über eine Leitung 9 mit einem Zulauf 8 eines Arbeitsbehälters 10 verbunden ist. Dieser Arbeitsbehälter 10 weist einen weiteren Zulauf 11 auf, der mit einem Konzentratablauf 12 einer Ultrafiltrationsanlage 13 verbunden ist.

Der Einlass 14 der Ultrafiltrationsanlage 13 ist mit einem Ablauf 15 des Arbeitsbehälters 10 verbunden. Der Permeatablauf 16 der Ultrafiltrationsanlage 13 ist über eine Leitung 17 mit dem Verbraucher 1 verbunden, wodurch das gereinigte Fluid dem Verbraucher 1 wieder zugeführt werden kann.

Der Arbeitsbehälter 10 weist einen weiteren Ablauf 18 auf, der über eine Leitung 19 mit einem Sedimentationsbehälter 20 verbunden ist. Dabei ist ein Sedimentablauf 21 dieses Sedimentationsbehälters 20 mit einer Zentrifuge 22 verbunden, der ein Konzentratbehälter 23 nachgeordnet ist.

Ein Klarablauf 24 der Zentrifuge 22 ist über eine Leitung 25 mit einem Einlauf des Vorsedimentationsbehälters 2 verbunden, in den auch eine an den Klarablauf 26 des Sedimentationsbehälters 20 angeschlossene Leitung 27 mündet. Dabei ist der Sedimentablauf 28 des Vorsedimentationsbehälters 2 mit einem Einlauf des Sedimentationsbehälters 20 verbunden.

Das mit Verunreinigungen beladene Fluid des Verbrauchers 1 gelangt in den Vorsedimentationsbehälter 2, in dem sich die leicht absetzbaren Verunreinigungen absetzen. Das vorgeklärte Fluid gelangt in den als Zwischenspeicher dienenden Lagerbehälter 6 und von diesem nach Bedarf in den Arbeitsbehälter 10. Aus diesem gelangt das Fluid in die Ultrafiltrationsanlage 13, in der das Fluid vollständig gereinigt und dem Verbraucher 1 wieder zugeführt wird.

Das aus der Ultrafiltrationsanlage 13 abgezogene Konzentrat wird dem Arbeitsbehälter 10 wieder zugeführt und auf diese Weise der Anteil an Konzentrat im Arbeitsbehälter 10 erhöht und in den Sedimentationsbehälter 20 abgezogen.

Der Sedimentationsbehälter 20 wird aus dem Vorsedimentationsbehälter 2 und dem Arbeitsbehälter 10 beschickt. In diesem setzen sich die Fremdstoffe des Fluids ab und werden der Zentrifuge zugeführt, in der es zu einer sehr weitgehenden Trennung von Fluid und Fremdstoffen kommt. Das in der Zentrifuge 22 abgeschiedene Fluid wird ebenso wie der Klarablauf 26 des Sedimentationsbehälters 20 dem Vorsedimentationsbehälter 2 zugeführt.

Das im Konzentratbehälter 23 gesammelte Konzentrat kann je nach Art des Konzentrats weiter behandelt werden. Beispielsweise kann ein in der Lebensmittelindustrie angefallenes Konzentrat, z.B. Stärke, mit UV-Licht entkeimt und weiter behandelt werden.

Es werden nun einige Beispiele angegeben.

Beispiel 1:

In einer typischen Anwendung einer Metallsäge fallen beispielsweise 16 m 3/h Sägewasser mit Metallspänen verunreinigt an. Diese Späne sind bei manchen Metallen so fein, dass sie nur mit einer Ultrafiltration zurückgehalten werden können. Bisher wurde solches Wasser schon mit verschiedenen Verfahren filtriert und teilweise zurückgewonnen, aber es entstand ein namhafter Abwasserstrom von etwa 15%, der klassisch entsorgt wurde.

Nach Anwendung des erfindungsgemäßen Verfahrens reduziert sich der Aufwand, erhöht sich die Rückgewinnungsrate und ergibt sich die Möglichkeit der Wiederverwendung des zerspanten Metalls.

In diesem Beispiel fallen 16 m³/h, beladen mit 0,8 g/l Metallspänen, an.

**Tabelle 1**

| | bisher | Erfindung |
|---|---|---|
| Permeat | 13,6 m³/h | 15,97 m³/h |
| Konzentrat aus UF | 2,4 m³/h | 0,125 m³/h |
| Rückgewinnung % | 85 | > 99 |
| Abfallstrom | 2,4 m³/h | 0 |
| Metallrückgewinnung | 0 | 25kg/h feucht |
| Chemikalien | Fällung/Flockung | 0 |

Unter Permeat wird hier der gesamte wieder gewonnene Fluidstrom verstanden; d.h. auch jener Teil des Konzentrats der Ultrafiltration, der durch die weiteren Stufen wieder gewonnen wird. Deshalb ist in der Spalte "Erfindung" die Summe von "Permeat" und "Konzentrat aus UF" höher als die zugeführte Menge.

Hier gelangen wegen des relativ sauberen Wassers feine Membranen zur Anwendung, die für Klarwasseranwendungen üblich sind, z.B. so genannte Hohlfasermembranen mit freiem Einlaufdruck oder besser Mehrkanal-Keramikmodule mit bis zu 6 bar Vordruck mit Kanalöffnungen bis zu 3 mm bei relativ geringer Anströmgeschwindigkeit von 2-6 m/s, vorzugsweise 4 m/s.

Die Anwendungstemperatur ist durch die Temperaturbeständigkeit des Membranmaterials begrenzt und kann von +5°C bis zu 95°C für Keramikmembranen reichen. Bevorzugt werden 25°C.

Die Erfahrung zeigt eine höhere erzielbare Rückgewinnungsrate mit keramischen Membranen gegenüber solchen auf organischer oder Zellulosebasis.

Wegen der hohen Differenz aus dem spezifischen Gewicht zwischen Flüssigkeit und Späne wird eine hohe Durchsatzleistung von bis zu 3 m³/h an der Zentrifuge erzielt, wobei die erzielbare Trockensubstanz unabhängig von der Temperatur hoch ist.

Beispiel 2:

In einer typischen Anwendung im Tiefdruck fallen laufend mit Druckfarbe kontaminierte Wässer an, die herkömmlicherweise gefällt und destilliert oder mit einer Ultrafiltration filtriert und destilliert werden, um einen festen Abfallstrom zu erzielen.

Nach Anwendung des erfindungsgemäßen Verfahrens reduziert sich der Aufwand an Energie, erhöht sich die Rückgewinnungsrate und senken sich die Entsorgungskosten.

In diesem Beispiel fallen 4 m³/h Abwasser mit 10 g/l Farbstoffen an.

**Tabelle 2**

| | bisher | Erfindung |
|---|---|---|
| Permeat | 3,88 m³/h | 3,95 m³/h |
| Konzentrat unbehandelt | 120 l/h | 0 |
| Konzentrat behandelt | 0 | 50 kg/h |
| % TS im Konzentrat | 33 % | 80 % |

Die Differenzmenge, im üblichen 2-3-schichtigen Betrieb einige m³ täglich, braucht nicht mehr kostenpflichtig entsorgt zu werden, wobei das unbehandelte Konzentrat als Flüssigkeit vorliegt, das behandelte Konzentrat als Feststoff mit bis zu 85% Trockensubstanz.

Der hohe und problematische Feststoffgehalt hat zur Verwendung von keramischen, vorzugsweise Al₂O₃, Membranen oder solchen auf Kohlenstoffbasis geführt, die mit einer aktiven Filterschicht aus Metalloxiden, insbesondere Zirkoniumoxid oder Titanoxid, versehen sind, die ein- oder mehrfach aufgebracht wurde. Der Kanaldurchmesser beträgt 2-8 mm, vorzugsweise 6 mm.

Die Anwendungstemperatur ist durch die Rohrleitungsmaterialien und die Inhaltsstoffe der Flüssigkeit begrenzt und liegt zwischen 10-70°C, vorzugsweise 55-60°C.

An einer Zentrifuge, im speziellen Fall einer horizontalen Dekanter-Zentrifuge, wurde wegen der geringen Unterschiede im spezifischen Gewicht und der feinen Dispersion der Inhaltsstoffe in der Flüssigkeit mit geringer Durchsatzleistung von lediglich 120-300 I/h bei hoher Trommeldrehzahl und niedriger Differenzdrehzahl gearbeitet.

Es sind Anwendungen mit organischen Rohrmembranen in der Praxis bekannt, die aber wegen des hohen Ersatzteilaufwandes und dem damit verbundenen Personalaufwand nicht empfohlen werden, obwohl sie erfindungsgemäß bei geringer Leistung funktionieren.

Die Zentrifugentemperatur stellt auch zum Teil die erzielbare Trockensubstanz (% TS) im Konzentrat ein, hängt auch von der Zwischenlagerung ab und sollte wegen Dampfbildung nicht über 85°C, vorzugsweise 20-55°C betragen.

## Patentansprüche

1. Verfahren zur Wiedergewinnung von mit Fremdstoffen beladenen wässrigen, neutralen, alkalischen, sauren oder enzymatischen Fluiden, z.B. aus der zerspanenden Bearbeitung von Metallen und Kunststoffen, oder von mit Farben und Lacken verunreinigten Lösungen oder von mit organischen Substanzen, wie z.B. Stärke, verunreinigten Fluiden, bei welchem Verfahren das mit Fremdstoffen beladene Fluid einer Filtration, vorzugsweise einer Ultrafiltration, unterworfen wird, und wobei das bei der Filtration gewonnene Konzentrat durch Zentrifugieren auf einen höheren Gehalt an Trockensubstanz gebracht wird und der Klarablauf (24) der Zentrifuge (22) wieder der Filtration zugeführt wird, **dadurch gekennzeichnet, dass** die Filtration eine Querstromfiltration ist, bei der das mit Fremdstoffen beladene Fluid von einem Arbeitsbehälter zur Querstromfiltrationsanlage (13) geführt und das Konzentrat der Querstromfiltrationsanlage (13) wieder zum Arbeitsbehälter (10) geführt wird, sodass das Fluid im Arbeitsbehälter (10) bis zu einer vorgegebenen Konzentration aufkonzentriert wird, wonach das Fluid aus dem Arbeitsbehälter (10) der Zentrifuge (22) zugeführt wird, deren Klarablauf (24) wieder dem Arbeitsbehälter (10) zugeführt wird.

2. Verfahren nach Anspruch 1, **dadurch gekennzeichnet, dass** das zu reinigende Fluid zunächst in einem Lagerbehälter (6) gesammelt wird und dem Arbeitsbehälter (10) erst dann zugeführt wird, wenn das Fluid im Arbeitsbehälter (10) die vorgegebene Konzentration erreicht hat und der Zentrifuge (22) zugeführt wurde, wobei auch der Klarablauf der Zentrifuge (22) dem Lagerbehälter (6) zugeführt wird.

3. Verfahren nach Anspruch 1 oder 2, **dadurch gekennzeichnet, dass** das Konzentrat der Zentrifuge (22) zur Wiedergewinnung von Wertstoffen, wie Metall oder Stärke, benutzt wird.

4. Verfahren nach einem der Ansprüche 1 bis 3, **dadurch gekennzeichnet, dass** dem gefilterten Fluid vor dem Rückführen in die Verwendung zur Verminderung des organischen Kohlenstoffgehalts Ozon zugeführt wird.

5. Verfahren nach einem der Ansprüche 1 bis 4, **dadurch gekennzeichnet, dass** das Konzentrat der Zentrifuge desinfiziert wird, beispielsweise mit UV-Licht.

6. Verfahren nach einem der Ansprüche 1 bis 5, **dadurch gekennzeichnet, dass** zwischen der Filtration und dem Zentrifugieren eine Sedimentation des Konzentrats der Filtration zwischengeschaltet ist und das dabei gewonnene Sediment zentrifugiert wird.

7. Verfahren nach Anspruch 6, **dadurch gekennzeichnet, dass** der Klarablauf (26) der zwischengeschalteten Sedimentation nach einer Vorsedimentation der Filtration zugeführt wird, wobei auch das zu reinigende Fluid dieser Vorsedimentation zugeführt wird und das Sediment der Vorsedimentation der Sedimentation zugeführt wird.

8. Einrichtung zur Durchführung des Verfahrens nach einem der Ansprüche 1 bis 7 mit einer Filtrationsanlage, vorzugsweise einer Ultrafiltrationsanlage (13), und mit einer Zentrifuge, bei welcher Einrichtung der Ablauf eines Verbrauchers (1) eines Fluids - gegebenenfalls über einen Lagerbehälter (6) - mit einem Zulauf (8) eines Arbeitsbehälter (10) in Verbindung steht, wobei ein Ablauf (15) des Arbeitsbehälters (10) mit dem Einlass (14) der Filtrationsanlage in Verbindung steht, **dadurch gekennzeichnet, dass** die Filtrationsanlage eine Querstromfiltrationsanlage ist, dass der Konzentratablauf (12) der Filtrationsanlage (13) mit einem weiteren Zulauf (11) des Arbeitsbehälters (10) verbunden ist und dass ein weiterer Ablauf (18) des Arbeitsbehälters (10) mit der Zentrifuge (22) in Verbindung steht.

9. Einrichtung nach Anspruch 8, **dadurch gekennzeichnet, dass** der Arbeitsbehälter (10) über einen Sedimentationsbehälter (20) mit der Zentrifuge (22) verbunden ist.

10. Einrichtung nach Anspruch 9, **dadurch gekennzeichnet, dass** die Klarabläufe (24, 26) der Zentrifuge (22) und des Sedimentationsbehälters (20) mit Einläufen eines Vorsedimentationsbehälters (2) verbunden sind, der dem Verbraucher (1) unmittelbar nachgeschaltet ist und dessen Klarablauf (4) - gegebenenfalls über den Lagerbehälter (6) - mit dem Arbeitsbehälter (10) verbunden ist und dessen Sedimentablauf (28) mit dem Sedimentationsbehälter (20) verbunden ist.
